# EUROPEAN PATENT APPLICATION

(11) **EP 2 363 066 A1**
(43) Date of publication of application: **07.09.2011**
(21) Application number: 11155424.2
(22) Date of filing: 22.02.2011
(51) Int. Cl.: A61B 5/11, A61C 19/04, A61B 6/03, A61B 6/14

(54) **Dental diagnosis system and dental care system**

(30) Priority: 03.03.2010 JP 2010047139
(71) Applicant: GC Corporation, Bunkyo-ku Tokyo 113-0033 (JP)
(72) Inventor: Shinjo, Takao, Tokyo 174-8585 (JP); Kinami, Ryoji, Tokyo 174-8585 (JP)
(74) Representative: Smart, Peter John

(57) **Abstract**

The present invention is to provide a dental diagnosis system which is capable of grasping detailed jaw motion information on a patient-by-patient basis. The dental diagnosis system comprises: an information gathering means comprising a jaw motion analyzer for measuring jaw motion and a computed tomography device; and an information processing means configured to carry out an operation, in accordance with the measurement results of the information gathering means, to combine a measurement result of jaw geometry obtained from the computed tomography device and a measurement result of jaw motion obtained from the jaw motion analyzer.

## Description

### Technical Field

The present invention relates to a dental diagnosis system based on jaw motion analysis and measurement of jaw geometry, and relates to a dental care system having the dental diagnosis system. More particularly, the invention relates to a dental diagnosis system which enables to make a diagnosis by accurately grasping symptom on a patient-by-patient basis and to treat the patient based on the diagnosis, and relates to a dental care system having the dental diagnosis system.

### Background Art

In the field of dentistry, jaw motion of a patient is analyzed and the data obtained from the analysis may be used for diagnosis and treatment for occlusion and defect of temporomandibular joint. To carry out the diagnosis and treatment, various jaw motion analyzers and analytical methods, for example, shown in Patent documents 1 and 2 are known.

Patent Document 1: Japanese Patent Application Laid-Open (JP-A) No. 2000-107207
Patent Document 2: JP-A No. 2005-349176

### Disclosure of the Invention

### Problems to be solved by the Invention

It is possible to obtain data of jaw motion by using the conventional jaw motion analyzers such as those of Patent documents 1 and 2. After analyzing the data, it is also possible to visually display the jaw motion based on the data by using a drawing of general skull.

However, in general, jaw motion varies depending on the inherent structure of patients' skull, condition of jaws, and experience of dental treatment and the type of the treatment. So, by using only the jaw motion data and/or the visual display of the jaw motion data simply shown on a drawing of general skull, these methods have limitations of grasping the detailed condition of each patient. Therefore, an adequate and accurate dental diagnosis by grasping more detailed jaw motion information on a patient-by-patient basis has been demanded.

Accordingly, an object of the present invention is to provide a dental diagnosis system which enables to grasp detailed jaw motion information on a patient-by-patient basis. Another object of the invention is to provide a dental care system comprising the dental diagnosis system and means for specific treatments.

### Means for Solving the Problems

Hereinafter, the present invention will be described. In order to make the understanding of the present invention easier, reference numerals of the attached drawings are quoted in brackets; however, the present invention is not limited by the embodiment shown in the drawings.

The first aspect of the present invention is a dental diagnosis system (10) comprising: an information gathering means (11) comprising a jaw motion analyzer (12) for measuring jaw motion and a computed tomography device (13); and an information processing means (18) configured to carry out an operation, in accordance with the measurement results of the information gathering means (11), to combine a measurement result of jaw geometry obtained from the computed tomography device (13) and a measurement result of jaw motion obtained from the jaw motion analyzer (12).

The second aspect of the invention according to the first aspect of the invention is characterized in that when combining the measurement result of jaw geometry obtained from the computed tomography device (13) and the measurement result of jaw motion obtained from the jaw motion analyzer (12), the operation is carried out while matching three or more coordinates or at least one reference plane selected from a three-dimensional space of the data of respective devices.

The third aspect of the invention according to the first or second aspect of the invention is characterized in that the information processing means (18) is configured to carry out image processing to display the results of operation as a three-dimensional image.

The fourth aspect of the invention according to any one of the first to third aspects of the invention is characterized in that the information gathering means (11) further comprises at least either one of an occlusal force measuring device (15) and a surface profile measuring device (16), and the information processing means (18) is configured to carry out operation to combine measurement results obtained from at least either one of the occlusal force measuring device (15) and the surface profile measuring device (16) with the measurement results of jaw geometry obtained from the computed tomography device (13).

The fifth aspect of the invention according to any one of the first to fourth aspects of the invention is characterized in that not only the measurement result obtained from the information gathering means (11) but also the shape information of the dental appliances can be inputted into the information processing means (18), and the information processing means (18) comprises a means configured to simulate at least jaw motions based on the measuring results obtained from the information gathering means (11) and shape information of the dental appliances.

The sixth aspect of the present invention is a dental care system (1) comprising: a dental diagnosis system (10) according to any one of the first to fifth aspects of the invention; and a means configured to receive an output signal from the dental diagnosis system and to fabricate a dental appliance based on the signal.

### Effects of the Invention

According to the present invention, it is possible to grasp detailed jaw motion information in accordance with the jaw structure on a patient-by-patient basis and possible to make an adequate diagnosis of various problems including jaw motions in accordance with respective patients' condition. In addition, it is also possible to obtain a means for adequate treatment based on the diagnosis results and respective patients' condition.

### Brief Description of the Drawings

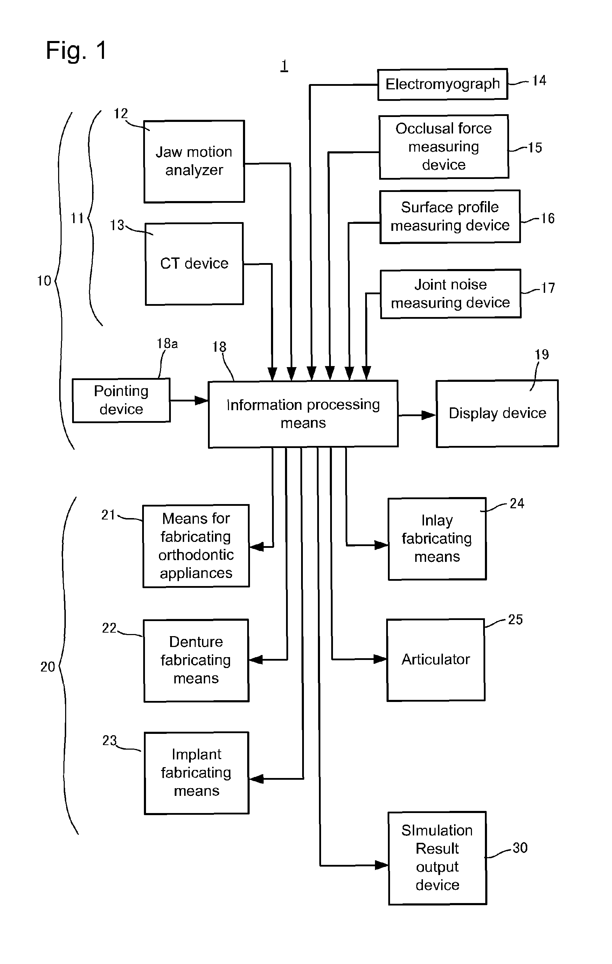

Fig. 1 is a schematic view of an embodiment of a dental diagnosis system and a dental care system including the dental diagnosis system.

### Description of Modes for Carrying Out the Invention

The functions and benefits of the present invention will be apparent from the following description of modes for carrying out the invention. Hereinafter, the invention will be described based on the embodiment shown in the drawings. However, the invention is not limited by these embodiment.

Fig. 1 is a schematic view of an embodiment of a dental care system 1 including a dental diagnosis system 10. The dental care system 1 comprises: a dental diagnosis system 10; a dental appliance fabricating means 20; and a simulation output device 30. Each of these elements will be described as below.

The dental diagnosis system 10 comprises: an information gathering means 11; an information processing means 18; and a display device 19. Here, the information gathering means 11 comprises: a jaw motion analyzer 12; a computed tomography (CT) device 13; a electromyograph 14; an occlusal force measuring device 15; a surface profile measuring device 16; and a joint noise measuring device 17.

The jaw motion analyzer 12 is a device for measuring patient's jaw motion; a known device can be used. The jaw motion analyzer 12 is a device to convert the jaw motion into numerical data by detecting movement of oral cavity and head in response to the opening and closing of the mouth by sensor. There are various types of jaw motion analyzer of which measurement sensor may be, for example, contact and noncontact sensors, or optical, magnetic, ultrasonic, and physical (displacement) sensors. In the dental diagnosis system 10 and the dental care system 1 of this embodiment, either one of the jaw motion analyzer may be used.
It should be noted that in view of carrying out accurate measurement, measurement using the jaw motion analyzer 12 is preferably carried out with three-dimensional (3D) six degree of freedom (DOF). The term "3D 6-DOF" means 3-DOF translation (X, Y, Z) and 3-DOF rotation (θx, θy, θz). These are essential degree of freedoms to carry out accurate motion analysis.

Examples of the CT device 13 maybe a dental CT device. The dental CT device is the one which enables to obtain results of 3D CT X-ray measurement with respect to the measuring object, i.e. tooth, jawbone, and jaw soft tissue; a known device can be used. Examples of a typical structure of the dental CT device comprises: an arm arranged in substantially horizontal manner; an X-ray irradiator set up on one end of the arm; and an X-ray receiver set up on the other end of the arm, wherein the arm can rotate in a horizontal plane. A patient is placed between the X-ray irradiator and the X-ray receiver; then, the X-ray irradiator and the X-ray receiver turn around the patient when the arm rotates. By carrying out X-ray irradiation and X-ray-receiving at each angular position of the turn, information (i.e. measurement result) of the measuring object (e.g. tooth) can be obtained.

The CT device 13 is used for obtaining X-ray tomography measurement information of each tooth; it may also comprise the so-called panorama measurement to obtain measurement results of the entire dental arch. The CT mode for obtaining measurement result of each tooth and a panorama mode which enables to obtain measurement result of dental arch are usually integrated in one device. However, the CT device is not necessarily limited to this type; namely, a device for the CT mode and a device for the panorama mode may be set up separately.

The electromyograph 14 is to measure myogenic potential; among the myogenic potential, this device is to measure myogenic potential of masticatory muscle. More specifically, myogenic potential sensors are mounted on a patient and then the electromyograph 14 can obtain data of myogenic potential at a time of mastication. As the electromyography, a known device can be used.

The occlusal force measuring device 15 is a device to measure patient's occlusal force; a known device can be used. An example of the occlusal force measuring device 15 comprises a film for occlusal force measurement which changes its color in response to different occlusal pressure, wherein when the film is inserted into the patient' s oral cavity and the patient bites the film, the color of the film changes, then, the expressed color is converted into numerical data as a concentration of the color by using, for example, charge coupled device (CCD) camera so as to obtain occlusal force as electrical signals.
Moreover, as an occlusal force measuring device 15, there may be a measurement device which uses piezo-electric element and measures pressure (occlusal force) in real-time.

The surface profile measuring device 16 is a device to obtain information of surface profile of oral cavity and head. As the device, for instance, conventional camera, intraoral camera, and surface profile measuring device 16 may be used. By using these devices, it is possible to obtain information of surface profile of oral cavity, tooth, and head. As the surface profile measuring device 16, a known device can be used.

The joint noise measuring device 17 is a device to detect noise generated when moving joint and the like. For example, in a case of temporomandibular arthrosis, noise is generated from temporomandibular joint by opening and closing the mouth. The noise is detected for making a diagnosis of symptom. Practically, an example of sensor to detect noise may be high-sensitivity condenser microphone. More specifically, a plurality of sensors are adhered on the surface of the patient's face to detect the noise.

Each device and equipment to be provided to the above information gathering means 11 is configured to eventually convert the obtained results into electronic or magnetic data and transmit the data to the information processing means 18.

The information processing means 18 is configured to receive measurement information from the information gathering means 11, to carry out an operation, and to provide operation results to the operator of the information processing means 18. As long as the means has the above features, structure of the means is not particularly limited. For example, there may be a means comprising: an input port which receives input information from the information gathering means 11; a ROM in which e.g. arithmetic expression and data are memorized; a RAM as work area of operation; a central processing unit (CPU) in which operation is carried out; and output port for outputting the signals of operation results.

Specifically, the information processing means 18 carries out operation to combine the results of patient's jaw motion obtained from the jaw motion analyzer 12 with the measurement results of patient's head (jaws) obtained from the CT device 13. In this operation, muscle movements obtained by electromyograph 14, occlusal force obtained by occlusal force measuring device 15, and/or each surface profile result obtained by surface profile measuring device 16 may also be combined for operation. By combining muscle movements obtained by electromyograph 14, occlusal force obtained by occlusal force measuring device 15, and/or each surface profile result obtained by surface profile measuring device 16 for the operation, it is possible to evaluate the measurement result of patient's jaw motion and e.g. occlusal force in accordance with the patient's actual jaw structure; thereby it is possible to make a highly accurate diagnosis.

The noise data obtained by the joint noise measuring device 17 can be integrated with other data by replaying the noise and synchronizing with the image data. By the method, together with the movement of temporomandibular joint and data from the electromyography, it is possible to improve certainty of diagnosis and to easily make the decision of therapeutic plan.

In the information processing means 18, data obtained from each device of the information gathering means 11 are integrated by matching three or more coordinates selected from a three-dimensional space, at least one reference plane, or a particular coordinate of data obtained from respective devices. The method for selecting such reference information (e.g. certain points) is not particularly limited. For example, there may be: a method using a pointing device 18a to specify the points; a method determining the above specific points, plane, or coordinates in advance as a reference; or a method to specify the above specific points, plane, or coordinates on a software each time.

When integrating the data, by aligning the scale of data derived from each device, it is possible to improve the accuracy of the output; so it is preferably that a discretionary scale can be specified. Therefore, information processing means 18 preferably comprises a means to adjust the scale automatically or manually.

In addition, when integrating the data, it is eventually necessary to carry out processing the data as 3D data. If the input from the information gathering means 11 is two-dimensional (2D) data, angular information at a time of acquisition of the 2D data and information of the reference plane (3D positional information) on the 3D data are provided at the same time, and the 2D data is integrated into the 3D data at a specified angle. That is, the 2D data preferably includes not only the mere 2D data but also angular information and positional information at a time of acquisition of the 2D data. Preferably, reference plane and coordinates for projecting the 2D data into the 3D data may be specified in the 2D data and scale marks for ensuring the accuracy of the scale may be given in the 2D data.
For instance, when integrating the tomographic 2D data obtained from CT image information, if a position to be the cross section is specified from the reference plane or coordinates at the time of integration and an image data derived from other information gathering means is integrated into the cross section, it is possible to more easily integrate the 2D data into the 3D data.

Other than the above, alternatively, by specifying the curved surface of the obtained 3D data and fitting the 2D data into the specified curved surface, the 2D data can also be integrated into the 3D data. In this case, three or more points in the 3D data are specified and the corresponding points of the 2D data are fitted into the points of the 3D data. In the method, so as to improve the accuracy of integration, when acquiring the 2D data, data about a particular position is acquired from a plurality of directions, and then the obtained data may be fitted into the 3D data.

Accordingly, by the information processing means 18 which integrates the information from the information gathering means 11, detailed jaw motion in accordance with the jaw structure can be grasped on a patient-by-patient basis and it is possible to make an adequate diagnosis of various problems including jaw motions in accordance with respective patients' condition. Moreover, it is possible to obtain a means for adequate treatment based on the diagnosis results and respective patients' condition.

Further, to the information processing means 18, shape information of dental appliances such as the below described orthodontic appliances and denture can be inputted. The information processing means 18 may also include a means for simulating motion of the jaws and the rest of the head when applying the orthodontic appliances, denture, and so on in the oral cavity. That is a means to simulate jaw motion and motion of head including oral cavity based on the measurement results obtained from the information gathering means 11 and shape information of the dental appliance. According to the means, it is not only possible to make a diagnosis and to make plans of treatment, but also possible to simulate the condition after treatment by inputting data to be expected after treatment. Therefore, it is possible to clearly accurately grasp the patient's condition after treatment; for example, the future troubles can be foreseen, which results in more appropriate treatment.

In other words, when examining how to apply the various appliances obtained by the dental appliance fabricating means to the patient, although the conventional method needs consideration based on 2D data or static data, dynamic data can be used with this invention. By using dynamic data, it is expected to figure out a cause of trouble, which is not possible with the static data. So, a simulation in accordance with the clarified cause of trouble makes it possible to determine shape of dental appliance which reflects the patient's dynamic condition.

Still further, with the highly accurate data thus obtained, it is not only possible to provide treatment for patient as ex-post facto support, but also possible to grasp the patient's current condition and point out problems in view of preventive effect.

In the information processing means 18, image processing for converting the obtained operation result into 3D image is preferably carried out. Hence, it is possible to display the image on the below-described display device 19.

The display device 19 is a means to show the information from the information processing means 18 in a form of image; examples thereof may be the so-called monitor. By the display device 19, it is possible to visually understand various information obtained by the information processing means 18; and the shown image can be an important source of information for an operator to make a diagnosis.

Hereinafter, the dental appliance fabricating means 20 will be described. The dental appliance fabricating means 20 is a means to fabricate various dental appliances required for treatment, based on the operation result of the information processing means 18. For example, there may be orthodontic appliances, denture, implant, inlay, and articulator. These dental appliances have slightly different shapes on a patient-by-patient basis; so, it is necessary to fabricate individually. If a doctor fails to provide an accurate dental appliance appropriate for an individual patient, trouble(s) may be caused by the dental appliance.
According to the dental care system 1, as described above, grasp of current condition and diagnosis are accurately performed in the dental diagnosis system 10 on a patient-by-patient basis; thus, by fabricating a dental appliance in accordance with the information, it is possible to provide an accurate and comfortable dental appliance to the patient.

Moreover, when a plurality of dental appliances need to be fabricated for a patient, since the original information is integrated in the information processing means 18, the accurate information can be easily extracted. In addition, it is also possible to prevent cause of mismatch among the plurality of dental appliances.

When fabricating the below-listed respective dental appliances, various data are provided by the information processing means 18. Method of data conversion into each fabrication means is not particularly limited; for example, in cases of inlay fabrication and orthodontic therapy those of which require manual works by dental technicians or dentists, numerical data for fabrication are provided. When fabricating inlay and implant upper structure by using CAD/CAM, numerical data corresponding to CAD/CAM are used. Conversion to e.g. articulator only requires output of positional and angular information for transferring jaw motion to the articulator.

Hereinafter, content of the dental appliance fabricating means 20 shown in Fig. 1 will be listed as an example.

The means for fabricating orthodontic appliances 21 is a means to fabricate an orthodontic appliance for treating malocclusion and jaw deformity. In the orthodontic treatment, teeth are moved and/or upper and lower jawbones undergo structural changes by orthodontic force; the appliance which imparts the orthodontic force is the orthodontic appliances.

The denture fabricating means 22 is a means to fabricate denture and partial denture.

The implant fabricating means 23 is a means to fabricate implant. Implant treatment is to plant and fix an implant as an artificial tooth in an area where the original tooth missing for some reason used to be located. Implant consists of an artificial root and an upper structure having a shape of tooth and being exposed in the oral cavity.

The inlay fabricating means 24 is a means to fabricate inlay as filling made of metal or ceramics to be fit in teeth.

The articulator 25 is a device which enables to reproduce various sorts of positions of jaw motion and occlusion on the model; a known articulator can be used. Articulator is classified particularly based on method (degree) of adjustment; e.g. the types include: average articulator, semi-adjustable articulator, and fully-adjustable articulator. In this embodiment, any one of articulators may be used.

Information required in each of these means for fabricating appliances is read out in response to the respective cases.

The simulation output device 30 will be described. As above, when simulation is carried out by the information processing means 18, the results are outputted as an image. So, examples of the simulation output device 30 may be a monitor. By the simulation output device 30, it is possible to visually understand the simulation results. The simulation output device 30 may serve also the display device 19.

By the above-described dental diagnosis system 10 and the dental care system 1 including the dental diagnosis system 10, it is possible to grasp the detailed jaw motion information in accordance with the jaw structure on a patient-by-patient basis and possible to make an adequate diagnosis of various problems including jaw motions in accordance with respective patients' condition. In addition, it is also possible to obtain a means for adequate treatment based on the diagnosis results and respective patients' condition.

In the embodiment of the present invention, although the above various devices are listed as the information gathering means 11 of the dental diagnosis system 10, devices of the information gathering means 11 are not limited to these devices; other devices and equipments may be added. In addition, the information gathering means 11 may at least comprise: a jaw motion analyzer 12 and a CT device 13, so that other devices and equipments are optional. It should be noted that larger number of devices provide a larger amount of information, which may result in more accurate image/data of condition of the patient.

The above has described the present invention associated with the practical and preferred embodiments thereof. However, the invention is not limited to the embodiments disclosed in the specification. Thus, the invention can be appropriately varied as long as the variation is not contrary to the subject substance and conception of the invention which can be read out from the claims and the whole contents of the specification. It should be understood that the dental diagnosis system and dental care system with such an alternation are included in the technical scope of the invention.

### Description of the Reference Numerals

- 1: dental care system
- 10: dental diagnosis system
- 11: information gathering means
- 12: jaw motion analyzer
- 13: computed tomography (CT) device
- 14: electromyograph
- 15: occlusal force measuring device
- 16: surface profile measuring device
- 17: joint noise measuring device
- 18: information processing means
- 19: display device
- 20: dental appliance fabricating means
- 21: means for fabricating orthodontic appliances
- 22: denture fabricating means
- 23: implant fabricating means
- 24: inlay fabricating means
- 25: articulator
- 30: simulation output device

## Claims

1. A dental diagnosis system comprising: an information gathering means comprising a jaw motion analyzer for measuring jaw motion and a computed tomography device; and an information processing means configured to carry out an operation, in accordance with the measurement results of the information gathering means, to combine a measurement result of jaw geometry obtained from the computed tomography device and a measurement result of jaw motion obtained from the jaw motion analyzer.

2. The dental diagnosis system according to claim 1, wherein when combining the measurement result of jaw geometry obtained from the computed tomography device and the measurement result of jaw motion obtained from the jaw motion analyzer, the operation is carried out while matching three or more coordinates or at least one reference plane selected from a three-dimensional space of the data of respective devices.

3. The dental diagnosis system according to claim 1 or 2, wherein the information processing means is configured to carry out image processing to display the results of operation as a three-dimensional image.

4. The dental diagnosis system according to any one of claims 1 to 3, wherein the information gathering means further comprises at least either one of an occlusal force measuring device and a surface profile measuring device, and the information processing means is configured to carry out operation to combine measurement results obtained from at least either one of the occlusal force measuring device and the surface profile measuring device with the measurement results of jaw geometry obtained from the computed tomography device.

5. The dental diagnosis system according to any one of claims 1 to 4, wherein not only the measurement result obtained from the information gathering means but also the shape information of dental appliances can be inputted into the information processing means, and the information processing means comprises a means configured to simulate at least jaw motion based on the measuring results obtained from the information gathering means and shape information of the dental appliances.

6. A dental care system comprising: a dental diagnosis system according to any one of claims 1 to 5; and a means configured to receive an output signal from the dental diagnosis system and to fabricate a dental appliance based on the signal.
